# EUROPEAN PATENT SPECIFICATION

(11) **EP 1 056 510 B1**
(45) Date of publication and mention of the grant of the patent: **11.05.2005**
(21) Application number: 98907507.2
(22) Date of filing: 23.02.1998
(51) Int. Cl.: A61N 1/08, A61N 1/378

(54) **RF COUPLED, IMPLANTABLE MEDICAL DEVICE WITH RECHARGEABLE BACK-UP POWER SOURCE**
IMPLANTIERBARE MEDIZINISCHE VORRICHTUNG MIT HOCHFREQUENZ-EINKOPPLUNG ZUM AUFLADEN DER ENERGIEQUELLE
DISPOSITIF MEDICAL IMPLANTABLE A COUPLAGE RF, DOTE D'UNE SOURCE D'ALIMENTATION DE SECOURS RECHARGEABLE

(43) Date of publication of application: 06.12.2000
(73) Proprietor: Medtronic, Inc., Minneapolis, Minnesota 55432-5604 (US)
(72) Inventor: BARRERAS, Francisco Jose, Sr., Miami, FL 33181 (US); JIMINEZ, Oscar, Coral Gables, FL 33134 (US)
(74) Representative: Matschnig, Franz, Dipl.-Ing.
(86) International application number: PCT/US1998/003105
(87) International publication number: WO 1999/042173

(56) References cited:
- DE-A- 19 617 102
- US-A- 3 195 540
- US-A- 4 134 408
- US-A- 5 411 537
- US-A- 5 713 939

## Description

### 1. Field of the Invention.

The present invention relates to an implantable medical device including a rechargeable back-up power source and a charging unit for recharging the back-up power source via RF coupling.

### 2. Description of the Prior Art.

The concept of using an implantable, electrically operated medical device for treating specific diseases or physical disorders is well known. Examples of implantable, electrically operated medical devices are: cardiac pacemakers which restore a sick human heart to a normal rhythm, neural simulators which control nerve or brain response (such as pain or epileptic seizures), infusion pumps for subcutaneously drug delivery (such as insulin pump), and diagnostic devices for monitoring a patient's condition.

With respect to all of these implantable, electrically operated devices, it is necessary to provide power to the device implanted below the skin. Since the medical device is subcutaneously implanted in the patient, the power source must supply electrical energy for a reasonable period of time in order to reduce further surgical trauma to the patient and financial cost to the medical provider.

Several examples of such previously proposed implantable devices are disclosed in the following U.S. Patents:

| U.S.Patent No. | Patentee |
|---|---|
| 3,942,535 | Schulman |
| 4,041,498 | Kelley at al. |
| 4,408,607 | Maurer |
| 4,441,498 | Nordling |
| 4,793,353 | Borkan |
| 5,279,292 | Baumann et al. |
| 5,314,453 | Jeutter |
| 5,314,457 | Jeutter et al. |
| 5,476,488 | Morgan et al. |
| 5,480,415 | Cox et al. |

A system with the features of the first part of claim 1 is known from DE-A-196 17 102.

### SUMMARY OF THE INVENTION

According to the present invention there is provided an RF coupled implantable medical system comprising: a transmitting unit; a receiving unit including an implantable, electrically operated, medical device; the transmitting unit including RF energy transmitting circuitry, RF signal receiving circuitry and first control circuitry coupled to the RF energy transmitting circuitry and to said RF signal receiving circuitry for controlling the amount of RF energy transmitted to the receiving unit; the receiving unit including RF energy receiving circuitry, RF signal transmitting circuitry, a rechargeable power supply coupled to the RF energy receiving circuitry and second control circuitry for adjusting the charging current flowing into the rechargeable battery coupled to the rechargeable power supply, to the RF energy receiving circuitry, to the RF signal transmitting circuitry and to the implanted medical device; and mode selection circuitry for setting the transmitting unit to operate in one of the following modes: 1) "RF only", 2) "battery only" or (3) "combination of both".

Further, if desired, circuitry can be provided in the receiving unit for measuring the charge level of the rechargeable battery and, upon sensing a fully charged battery, automatically up-linking a coded signal which commands the transmitting unit to "stop" transmitting RF energy.

Further preferred features are defined in the dependent claims.

### BRIEF DESCRIPTION OF THE DRAWINGS

FIG. 1 is a block electrical, schematic circuit diagram of the overall system including a transmitting unit and an implanted receiver unit configured for an implantable, rechargeable tissue stimulator system.
FIG. 2A is a block electrical schematic circuit of a digital to analog converter in the receiver unit
FIG. 2B is a detailed electrical schematic circuit diagram of the digital to analog converter with current output shown in FIG. 2A which is used, under control of a micro controller, to regulate the constant current rate for recharging the back-up power source within the implanted receiver unit.
FIG. 3 is a block electrical schematic circuit diagram of the overall system as configured for an implantable, rechargeable drug delivery system.
FIG. 4 is a block electrical schematic circuit diagram of the overall system as configured for an implantable, rechargeable cardiac pacemaker system.
FIG. 5 is a block electrical schematic circuit diagram of the overall system as configured for an implantable, rechargeable cardioverter/defibrillator.
FIG. 6 is a block electrical schematic circuit diagram of the overall system as configured for an implantable, rechargeable monitor and diagnostic system.

### DESCRIPTION OF THE PREFERRED EMBODIMENT(S)

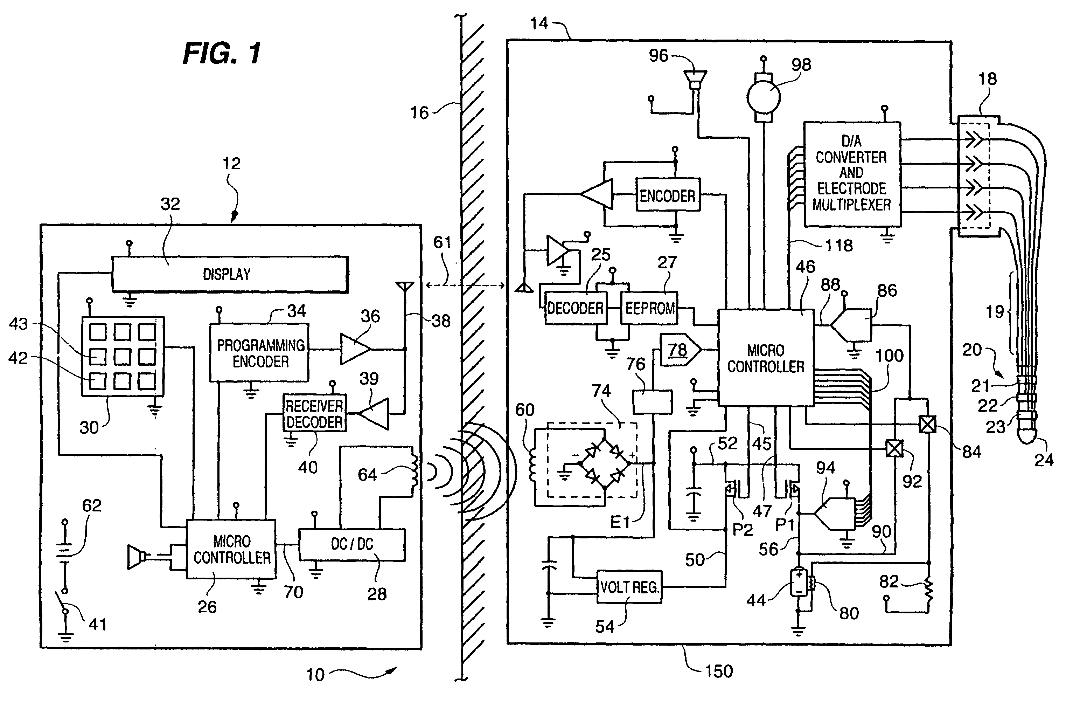

Referring to the drawings in greater detail, there is illustrated in FIG. 1 a block electrical schematic circuit diagram of an implantable, rechargeable tissue stimulator system 10. The system 10 includes a transmitter 12 and a receiver 14, the latter being surgically implanted beneath a patient's skin 16. The receiver 14 is connected, via lead connector 18, to an implanted medical device, which in this embodiment, is an implanted lead 19 which contains, at it's distal end 20, stimulating electrodes 21-24. These electrodes 21-24 are implanted adjacent to the target tissue to be stimulated (i.e., a specific nerve or a nerve bundle, a specific area of the brain or a specific muscle within the human body). The implanted receiver 14 receives therapy values, transmitted by the transmitter 12 via RF signals, which are decoded by decoder 25 and then stored in a non-volatile memory (EPROM) 27.

Referring again to FIG. 1, the major components of the transmitter 12 are a micro controller 26 which is used, via software, to: 1) control the output of a programmable DC to DC converter 28 in order to regulate the amount of RF energy to be coupled into the receiver 14; 2) read data and command inputs inputted via a keyboard 30, display messages and menus via a display 32, transmit therapy parameter values, via a programming encoder 34, a transmit driver 36 and an antenna 38, to the implanted receiver 14; 3) and receive commands and patient's diagnostic data, transmitted from the implanted receiver 14, via the antenna 38, an amplifier 39 and a decoder 40.

As shown in FIG. 1, when the transmitter 12 is powered up via a switch 41 and a "start therapy" key 42 on the keyboard 30 is pressed, the transmitter 12 will transmit the "start" command to the receiver 14 which will initiate delivery of therapy by the receiver 14. Likewise, if a "stop therapy" key 43 on the keyboard 30 is pressed, the transmitter 12 will transmit the "stop" command to the receiver 14 which will cease delivery of therapy.

The implanted receiver 14 of FIG. 1 can be programmed by the physician or patient to obtain it's operating power from one of three sources: 1) RF coupled energy only; 2) back-up rechargeable power supply/source 44 only; or 3) a combination of both whereby the implanted receiver 14 alternates automatically from one to the other according to a preset schedule programmed via the transmitter 12.

When "RF only" is selected from a menu displayed by the display 32 of the transmitter 12, an output port 45 of a micro controller 46 in the receiver 14 is switched to a "0" and a port 47 is switched to a "1" which places pmos transistor P2 in a conducting state and pmos transistor P1 in a non-conducting state. This effectively connects a line conductor 50 to a line conductor 52, making VDD equal to the output of a voltage regulator 54 which is at + 3.0 vdc.

When "battery only" is selected from the same menu, the output port 47 is switched to a "0" and the port 45 is switched to a "1", thus effectively connecting line conductor 56 to line conductor 52 and making VDD equal to the voltage level at the rechargeable power source 44.

As shown in FIG. 1, when "combination" is selected from the same menu, the system 10 will automatically switch the source of VDD to the output of the voltage regulator 54 (line conductor 50) when the transmitter 12 is proximal to the receiver 14, or to the rechargeable power source 44 when the transmitter 12 is removed away from the receiver 14. The automatic switching is performed by the micro controller 46 in response to the state of line conductor 50 which is at +3.0 volts when RF energy is being coupled into an inductor 60 (the transmitter 12 is proximal to the receiver 14) or is 0 volts in the absence of RF energy (the transmitter 12 is away from the receiver 14).

When the receiver 14 is programmed to "battery only" power acquisition mode, it's exclusive source of operating power becomes the rechargeable power source 44. After prolonged use, the rechargeable power source 44 will reach a near depleted level, at which point the receiver 14 will transmit, via an RF communication link 61, a "recharge" command to the transmitter 12. This will cause the transmitter 12 to generate, via the battery 62, the DC/DC converter 28 and an output inductor 64, high energy RF waves which are coupled into the inductor 60 contained within the receiver 14. The actual level of RF energy generated by the inductor 64 is regulated by an output port 70 of the micro controller 26 as a real-time response to data transmitted by the receiver 14 via the micro controller 46, the data representing the voltage level E1 at the output of the rectifier 74 in the receiver 14 which is measured via an attenuator 76 and an analog to digital converter 78. This feedback system extends the life of the battery 62 within the transmitter 12, by adjusting, as a function of distance between the inductors 64 and 60, the RF energy required to quickly recharge the rechargeable power source 44. A close proximity requires much less RF energy to recharge the rechargeable power source 44 than a longer distance would, in the same time. During this recharging operation, the micro controller 46 regulates, as a function of temperature, the current level used to recharge the rechargeable power source 44. The temperature is measured by a thermistor 80 which is adhered to the rechargeable power source 44 during manufacturing. The junction between the thermistor 80 and a resistor 82 form a voltage divider which is fed through an analog switch 84 to an analog to digital converter 86 and, via a line conductor 88, to the micro controller 46. As the voltage rises, the ohmic value of the thermistor 80 drops proportionally to the temperature, thus reducing the voltage at the line conductor 88 to the micro controller 46. This loop forms a temperature-controlled, current-regulated charging system which restricts the temperature rise of the rechargeable power source 44 during recharging, thus preventing the power source 44 from suffering electrolyte starvation and gas generation. Both of these phenomena will, if left unchecked, dramatically reduce the reliability and service life of the power source 44. Also, during recharging of the power source 44, the micro controller 46 will monitor the voltage level of the power source 44 via a line conductor 90, analog switch 92, the A/D converter 86 and, finally, the line conductor 88. Upon sensing a fully charged state, the micro controller 46 will telemeter to transmitter 12, via the RF communications link 61, a "stop" recharging command and simultaneously will turn off a D/A converter 94 which will cut off the current needed to charge the rechargeable power source 44. In this manner, the power source 44 cannot be overcharged, even if the "stop" command was not received by the transmitter 12 due to electromagnetic interference.

Referring to FIG. 1, when the receiver 14 is programmed to "RF only", the power acquisition mode, it's exclusive source of operating power is the low level RF energy generated by transmitter 12 and coupled into the inductor 60 within the receiver 14. The actual level of RF energy generated by the inductor 64 is regulated by the output port 70 of the micro controller 26 to the minimum level required to operate the receiver 14 and the rechargeable power source 44 is trickle charged, as a real-time response to data transmitted by the receiver 14 via the micro controller 26, i.e., the data representing the voltage level E1 at the output of the rectifier 74 which is measured via the attenuator 76 and the analog to digital converter 78. This feedback system extends the life of the battery 62 within the transmitter 12, by adjusting the RF energy required to operate the receiver 14 and maintain a trickle charge to the rechargeable power source 44, as a function of the distance between the inductors 64 and 60. At close proximity, much less RF energy is required to accomplish these functions than at a longer distance.

During trickle charging, the micro controller 46 regulates, as a function of temperature, the current level used to trickle charge the power source 46, by the same method already explained in the previous paragraph. Again, this prevents electrolyte starvation and gas generation within the rechargeable power source 44. Also, during trickle charging, the micro controller 46 will monitor the charge level of the power source 44, and upon sensing a fully charged state, the receiver 14 will telemeter to the transmitter 12 the "stop" recharging command and simultaneously will turn off the D/A converter 94 which will cut off the current needed to charge the power source 44. In this manner, the power source 44 cannot be overcharged, even if the "stop" command was not received by the transmitter 12 due to electromagnetic interference.

When the receiver 14 is programmed to "combination" power acquisition mode, the micro controller 46 will automatically switch delivery of operating power to the receiver 14 to RF coupled energy upon detection of RF induced voltage at E1. Likewise, the micro controller 46 will switch delivery of operating power to the rechargeable power source 44 upon loss of RF induced voltage at E1. The patient may select, via a menu shown in the transmitter's display 32, fast or trickle charge to the rechargeable power source 44.

Upon sensing that the charge in the rechargeable power source 44 is below a predetermined level, the micro controller 46 signals the patient, via an audible alarm 96 and/or a vibrating alarm 98, that the rechargeable power source 44 should be recharged.

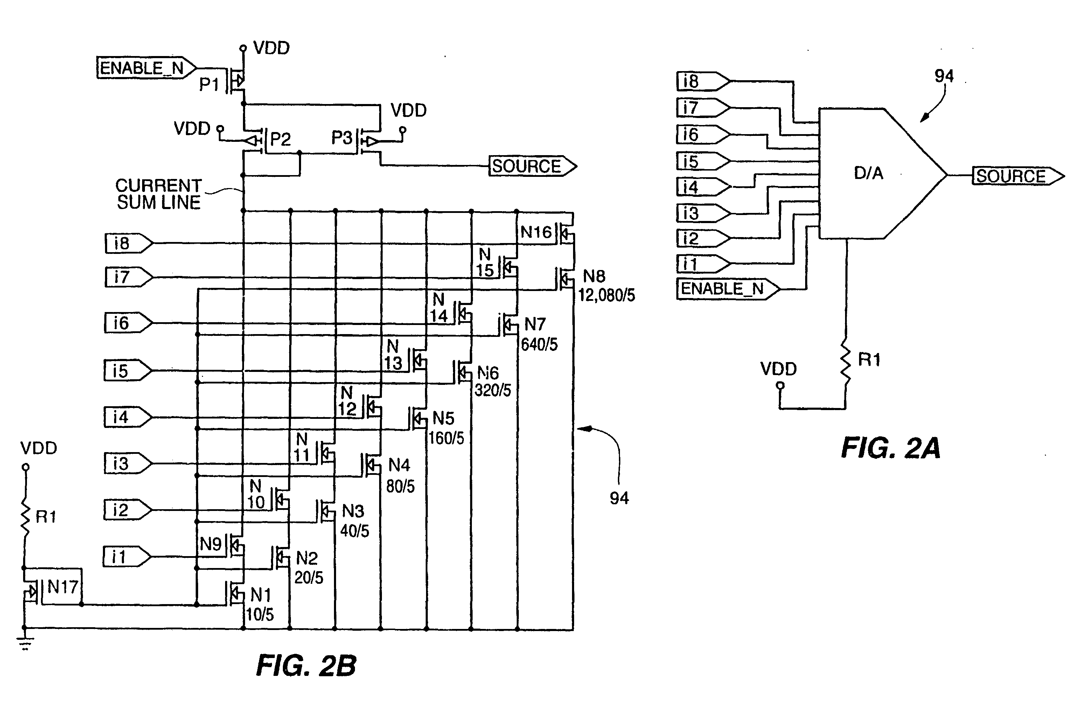

Referring to FIG. 2B, a detailed electrical schematic of the digital to analog (D/A) converter 94 is provided. The D/A converter 94 is software programmable, precision current source whose output is regulated by the micro controller 46. It should be noted that this type of D/A converter is best implemented into an integrated circuit where the electrical characteristics of the transistors can be precisely matched.

Referring again to FIG. 2B, resistor R1 is used to set the base bias current for the converter 94. Transistors N17 and N1 form a 1:1 current mirror where the current into the transistor N17 equals the current through the transistor N1, since both transistors have equal channel width and length. However, the channel width for the transistors N2 through N8 are binary weighted, so that the transistor N2 has twice the width of the transistors N1, N3 has twice the width of N2, and so forth. This binary scaling results in transistor N2 conducting twice the current of transistor N1 (assuming equal bias current), the transistor N3 conducting twice the current of the transistor N2, and so forth. The transistors N9 through N16 are used as pass devices to allow the current available at the transistor N1 through the transistor N8 to pass to the current sum line 1. The on-off state for the transistors N9 through N16 is governed by inputs i1 through i8 which, in one embodiment, are output ports from the micro controller 46 (bus line 100 in FIG. 1). In this manner, the micro controller 46 is able to select any value of current between 1 and 256 times that of the current flowing through the resistor R1 (bias current) to pass to the current sum line 1.

Input line "enable.n" is used to turn on and off the D/A converter. When "enable.n" is a "0", the transistor P1 conducts connecting VDD to the sources of the transistors P2 and P3 which form a 1:1 current mirror. Therefore, the current source by the transistor P3 equals the current flowing through the transistors P2, P3 being the output current device ("iSOURCE") for this D/A converter 94.

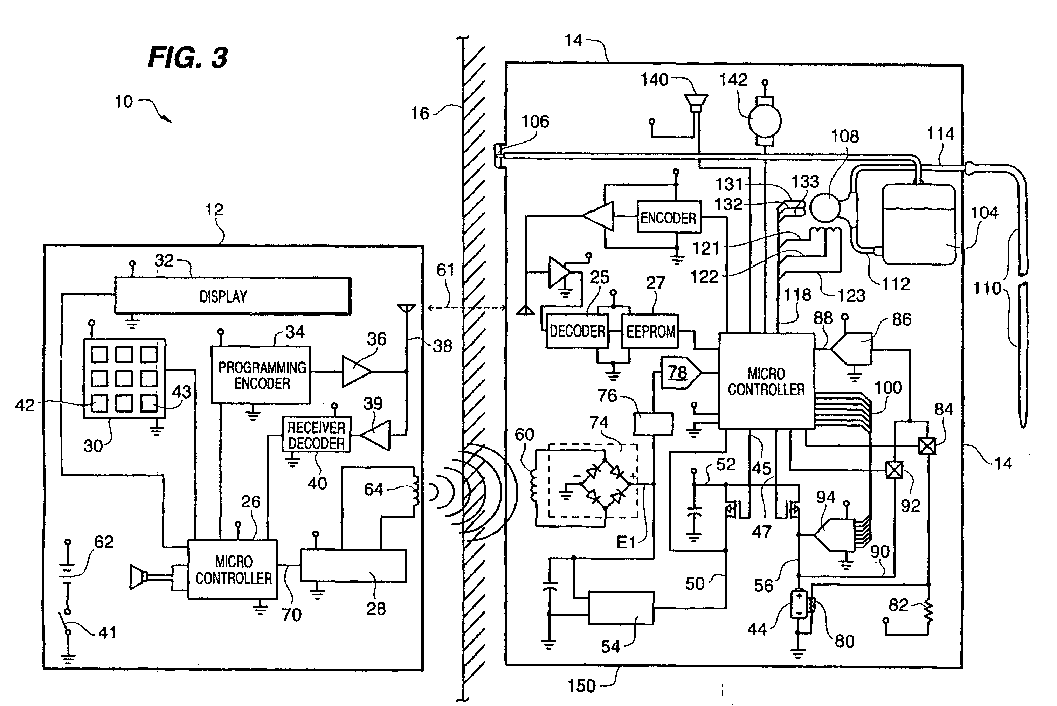

In FIG. 3 is shown a block diagram for an implantable, rechargeable drug delivery system. The block diagram for the transmitter 12 is the same as for the transmitter 12 shown in FIG. 1.

The block diagram for the receiver 14 contains the same power supply system, supply switching means and method for recharging the rechargeable power source 44 that has been already described above in connection with the description of FIG. 1, but has been modified to incorporate the components required to assemble an implantable drug delivery system. These components are: 1) a drug reservoir 104 which contains the drug to be delivered by the receiver 14; 2) a refill septum 106 used to percutaneously, via a hypodermic needle, refill the drug reservoir 104; 3) a portioning pump 108 used to dispense a precise volume of drug to a catheter 110 by making one or more small injections (portions); 4) a pump inlet tube 112; 5) a pump outlet tube 114; 6) the drug delivery catheter 110 which is used to carry and deliver to the target tissue the drug volume dispensed by the pump 108; and 7) a multi-wire cable 118 which carries the electrical signals for driving the pump 108.

Referring again to FIG. 3, the wire conductors 121-123 are used to drive the pump 108 and the wire conductors 131-133 are used to sense when a portion has been delivered. The micro controller 46 measures the time required for delivering each drug portion, and based on this time determines if the pump 108 is empty or contains fluid, since the former condition results is a faster time than the latter. Upon sensing a "pump empty" condition, the micro controller 46 signals the patient, via an audible alarm 140 and/or a vibrating alarm 142, that the reservoir 104 is empty. It should be noted that the titanium housing 150 of the receiver 14 should be in close proximity to the audible alarm 140 in order to transmit the sound waves to outside the human body.

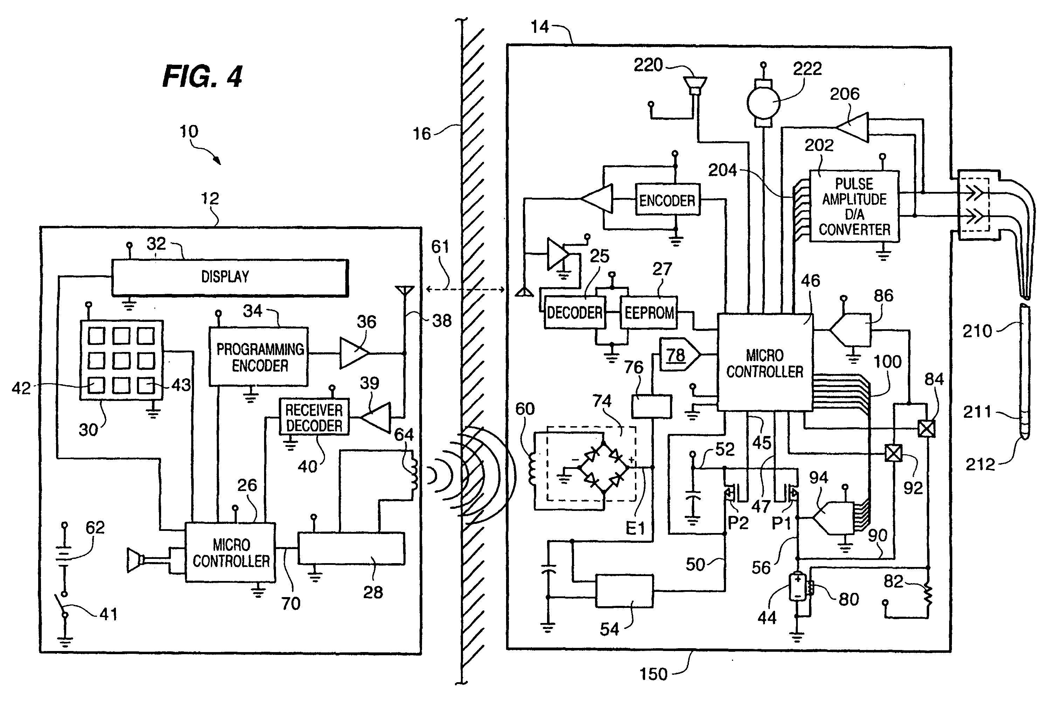

FIG. 4 is a block diagram of an implantable, rechargeable cardiac pacemaker system. The block diagram for the transmitter 12 is the same as for the block diagram of the transmitter 12 shown in FIG. 1.

The block diagram for the receiver 14 contains the same power supply system, supply switching means and method for recharging the rechargeable power source 44 that already have been described above in connection with the description of FIG. 1, but has been modified to incorporate the components required to assemble an implantable, rechargeable cardiac pacemaker system. These components are: 1) a pulse amplitude D/A converter 202 which is used to regulate, under command of the micro controller 46, the amplitude of the stimulating pulses delivered to the human heart; 2) a bus 204 which carries the binary value for the amplitude from the micro controller 46 to the D/A converter 202; 3) an amplifier and filter 206 which detects and amplifies the cardiac depolarization waves (such as R or P waves) and filters out other signal frequencies not related to cardiac activity; 4) an implanted lead 210 containing electrodes 211-212 which is used to deliver stimulating pulses to the heart in order to regulate the heart's rhythm, but which is used also to pick-up and carry the cardiac depolarization waves to the amplifier/filter 206. These cardiac waves are used by the micro controller 46 to measure the intrinsic rate of the heart. The measurement is utilized to determine if electrical stimulation pulses are needed to speed-up the heart.

Upon sensing that the charge in the rechargeable power source 44 is below a predetermined level, the micro controller 46 signals the patient, via an audible alarm 220 and/or a vibrating alarm 222, that the rechargeable power source 44 should be recharged.

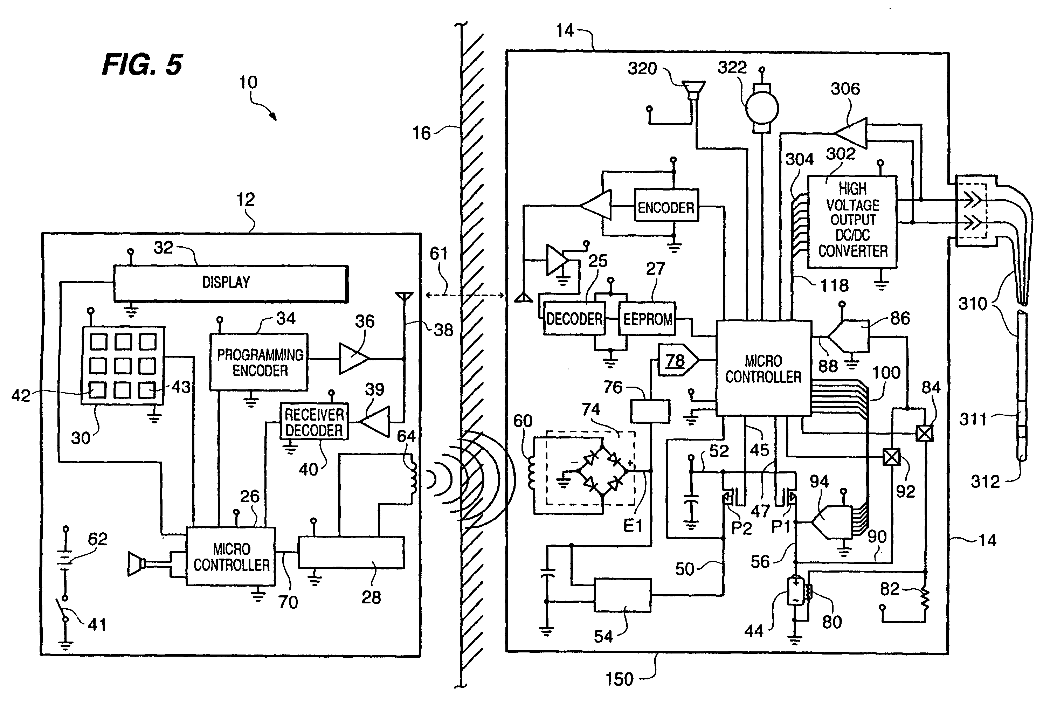

In FIG. 5 is illustrated a block diagram of an implantable, rechargeable cardioverter/defibrillator. The block diagram for the transmitter 12 is the same as the block diagram for the transmitter 12 shown in FIG. 1.

The block diagram for the receiver 14 contains the same power supply system, supply switching means and method for recharging the rechargeable power source 44 that already have been described above in connection with the description of FIG. 1, but has been modified to incorporate the components required to assemble an implantable, rechargeable cardioverter/defibrillator system. These components are: 1) a high voltage output, DC to DC converter 302 used to convert the low voltage available at the rechargeable power source 44 to a relatively higher voltage required to cardiovert a fibrillating human heart; 2) a bus 304 which carries the binary value for the voltage amplitude from the micro controller 46 to the D/A converter 302; 3) an amplifier/filter 306 used to detect the presence of a cardiac arrhythmia such as fibrillation or tachycardia; and, 4) an implanted cardioverting lead 310 containing cardioverting electrodes 311-312.

As shown in FIG. 5, the DC/DC converter 302 is used to generate either low voltage pulses to pace the human heart when needed or high voltage pulses to shock a large number of cardiac cells into synchrony, thereby restoring a normal cardiac rhythm. The micro controller 46, via the bus 304, regulates the timing and amplitude of low voltage pulses or high voltage shocks, depending if an arrhythmia is detected or not. Upon sensing that the charge in the rechargeable power source 44 is below a predetermined level, the micro controller 46 signals the patient, via an audible alarm 320 and/or a vibrating alarm 322, that the rechargeable power source 44 should be recharged.

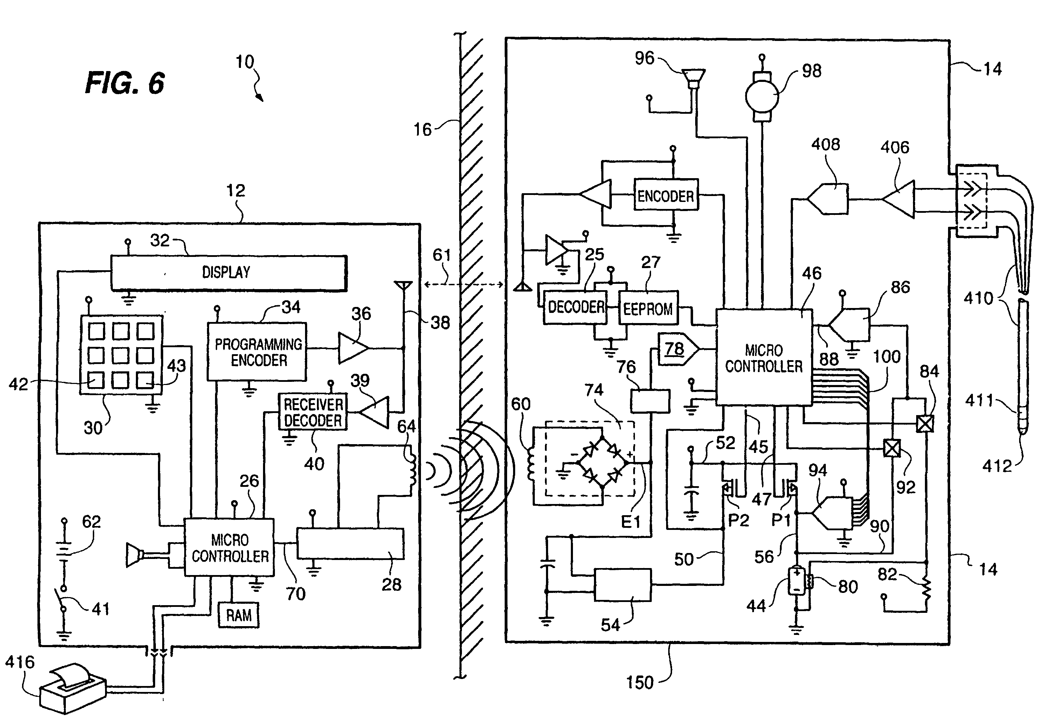

In FIG. 6 there is illustrated a block diagram of an implantable, rechargeable monitor and diagnostic system. The block diagram for the transmitter 12 is the same as the block diagram for the transmitter 12 shown in FIG. 1.

The block diagram for the receiver 14 contains the same power supply system, supply switching means and method for recharging the rechargeable power source 44 that already have been described in connection with the description of FIG. 1, but has been modified to incorporate the components required to assemble an implantable, rechargeable monitor and diagnostic system. These components are: 1) an amplifier/filter 406 used to amplify the desired biological signals and to filter out other undesirable signals; 2) an analog to digital converter 408 which is used to convert the biological signal into a digital value representative of frequency and amplitude of the biological signal; 3) a monitoring lead 410 containing electrodes 411-412 which are used to pick-up and carry the biological signals to the amplifier/filter 406.

The mission of the monitor and diagnostic system shown in FIG. 6 is to monitor and record, in a non-volatile memory 414, specific biological signals and events occurring adjacent to the monitoring electrodes 411-412. Later, at a convenient time, these recordings can be telemetered to the transmitter 12 which will produce, via a graphic recorder 416, a hard copy of the biological signals for the physician's examination and eventual diagnosis. Any time biological signals occur, they are scrutinized by the micro controller 46 for specific morphology which would cause the event to be stored into the memory 27 for later examination by the physician. An example of a typical use, would be to record dysfunctional endocardiac signals which, when inspected by a trained physician, may reveal the origin of a cardiac dysfunction not detected by conventional means, such as a surface EKG.

From the foregoing description, it will be apparent that the RF coupled, implantable medical system 10 with the rechargeable back-up power supply/source 44 of the present invention has a number of advantages, some of which have been described above and others of which are inherent in the invention. Also it will be understood that modifications can be made to the RF coupled, implantable medical system including the rechargeable back-up power supply/source 44 described above without departing from the teachings of the present invention. Accordingly, the scope of the invention is only to be limited as necessitated by the accompanying claims.

## Claims

1. An RF coupled implantable medical system (10) comprising:
a transmitting unit (12);
a receiving unit (14) including an implantable, electrically operated, medical device;
said transmitting unit including RF energy transmitting means (64), RF signal receiving means (38) and first control means (26) coupled to said RF energy transmitting means and to said RF signal receiving means for controlling the amount of RF energy transmitted to said receiving unit;
said receiving unit including RF energy receiving means (60), RF signal transmitting means, a rechargeable power supply (44) coupled to said RF energy receiving means and second control means (46) for adjusting the charging current flowing into said rechargeable battery coupled to said rechargeable power supply, to said RF energy receiving means, to said RF signal transmitting means and to said implanted medical device; and
mode selection means for setting said transmitting unit to operate in one of the following modes: 1) "RF only", 2) "battery only" or 3) "combination of both".

2. The system of claim 1 wherein said receiving unit, when said transmitting unit is set to operate in said "RF only" mode, is operable to supply electrical energy to said implantable device, so long as said transmitting unit is located proximate to said receiving unit and said receiving unit is sensing transmitted RF energy.

3. The system of claim 1 wherein said receiving unit, when said transmitting unit is set to operate in said "battery only" mode, is operable, periodically, to supply electrical energy to said implantable device from said rechargeable power supply for a period of at least 24 hours.

4. The system of claim 1 wherein said RF energy transmitting means of said transmitting unit includes mode selection means for recharging said rechargeable battery at a "fast" rate or at a "trickle" rate.

5. The system of claim 1 wherein said receiving unit, when said transmitting unit is set to operate in said "combination" mode, is operable to supply electrical energy to said implantable device through a rectifier directly to said implanted medical device, so long as said transmitting unit is located proximate to said receiving unit, and, separately, to "trickle charge" said rechargeable power supply.

6. The system of claim 1 wherein said receiving unit further comprises means for measuring the charge level of said rechargeable battery and, upon sensing a fully charged battery, automatically up-linking a coded signal which commands said transmitting unit to "stop" transmitting RF energy.

7. The system of claim 1 wherein said receiving unit includes a titanium housing enclosing said RF energy receiving means, said RF signal transmitting means, said rechargeable battery and said second control means.

8. The system of claim 1 wherein said RF energy transmitting means of said transmitting unit is constructed to transmit energy at a frequency as low as 10 Hz and up to at least 20,000 Hz.

9. The system of claim 1 wherein said rechargeable battery has a temperature sensor which is mounted closely adjacent thereto and which is coupled via said RF signal transmitting means to said first control means of said transmitting unit whereby the level of transmitted RF energy can be reduced proportionally to the reduction in charging rate of the rechargeable battery in said receiving unit, in order to reduce the power consumption from a power source powering said transmitting unit.

10. The system of claim 1 wherein said first control means of said transmitting unit includes means for controlling the level of RF energy transfer from the transmitting unit to the receiving unit relative to one or more of the following parameters; (a) the charge level of said rechargeable battery, (b) selected charging rate and (c) the selected power supply for said receiving unit.

11. The system of claim 1 wherein said transmitting unit includes a visual display coupled to said first control means.

12. The system of claim 1 wherein said transmitting unit includes a keyboard coupled to said first control means including keys to start' and stop recharging of said rechargeable battery within the implantable medical device.

13. The system of claim 1 wherein said transmitting unit includes a battery, whereby said transmitting unit is portable and not dependent upon an a.c. power source.

14. The system of claim 1 wherein said implantable medical device is selected from the group consisting of a tissue stimulator, a drug delivery system, a cardiac pacemaker system, and a cardioverter/defibrillator.

## Patentansprüche

1. Implantierbares, HF-gekoppeltes medizinisches System (10), welches aufweist:
eine Sendeeinheit (12);
eine Empfangseinheit (14) mit einer implantierbaren, elektrisch betriebenen medizinischen Einrichtung;
wobei die Sendeeinheit ein HF-Energie Sendemittel (64), ein HF-Signal Empfangsmittel (38) und ein erstes Steuermittel (26) besitzt, das mit dem HF-Energie Sendemittel sowie mit dem HF-Signal Empfangsmittel in Verbindung steht, um den Betrag der an die Empfangseinheit gesendeten HF-Energie zu steuern,
die Empfangseinheit ein HF-Energie empfangendes Mittel (60), ein HF Signal sendendes Mittel, eine wieder aufladbare Stromversorgung (44), welche mit dem HF-Energie Empfangsmittel verbunden ist, und ein zweites Steuermittel (46) zur Einstellung des in die wieder aufladbare Batterie fließenden Ladestroms aufweist, und das mit der wieder aufladbaren Stromversorgung, dem HF-Energie Empfangsmittel, dem HF-Signal Empfangsmittel und der implantierten medizinischen Einrichtung in Verbindung steht; und
ein Betriebsarten-Auswahlmittel, um die Sendeeinheit so einzustellen, dass sie in einer der folgenden Betriebsarten arbeitet: 1) "nur HF", 2) "nur Batterie" oder "Kombination von beiden".

2. System nach Anspruch 1, bei welchem für den Fall, dass die Sendeeinheit so eingestellt ist, dass sie in der Betriebsart "nur HF" arbeitet, die Empfangseinheit so lange in einem Betrieb zum Zuführen elektrische Energie an die implantierbare Einrichtung arbeitet, solange sich die Sendeeinheit in Nähe der Empfangseinheit befindet und die Empfangseinheit gesendete HF-Energie feststellt.

3. System nach Anspruch 1, bei welchem für den Fall, dass die Sendeeinheit so eingestellt ist, dass sie in der Betriebsart "nur Batterie" arbeitet, die Empfangseinheit an die implantierbare Einrichtung periodisch in einem Betrieb zum Zuführen elektrische Energie an die implantierbare Einrichtung arbeitet, um elektrische Energie aus der wieder aufladbaren Stromversorgung an die implantierbare Einrichtung für eine Periode von mindestens 24 Stunden zu liefern.

4. System nach Anspruch 1, bei welchem das HF-Energiesendemittel der Sendeeinheit ein Betriebsarten-Auswahlmittel aufweist, um die wieder aufladbare Batterie mit einer Rate "schnell" oder "sickernd" zu laden.

5. System nach Anspruch 1, bei welchem für den Fall, dass die Sendeeinheit so eingestellt ist, dass sie in der Betriebsart "Kombination" arbeitet, die Empfangseinheit in einem Betrieb zum Zuführen elektrischer Energie an die implantierbare Einrichtung über einen Gleichrichter direkt an die implantierte medizinische Einrichtung arbeitet, solange sich die Sendeeinheit in Nähe der Empfangseinheit befindet und, getrenntenfalls, die wieder aufladbare Stromversorgung "sickernd zu laden".

6. System nach Anspruch 1, bei welchem die Empfangseinheit weiters ein Mittel zum Messen des Ladepegels der wieder aufladbaren Batterie aufweist und bei Feststellen einer voll geladenen Batterie automatisch ein codiertes Signal abgibt, welches der Sendeeinheit befiehlt, das Senden von HF-Energie zu "stoppen".

7. System nach Anspruch 1, bei welchem die Empfangseinheit ein Titangehäuse besitzt, welches das HF-Energieempfangsmittel, das HF-Signal Sendemittel, die wieder aufladbare Batterie und das zweite Steuermittel umschließt.

8. System nach Anspruch 1, bei welchem das HF-Energie Sendemittel der Sendeeinheit dazu eingerichtet ist, Energie mit einer Frequenz hinunter bis 10 Hz und hinauf bis zumindest 20.000 Hz zu senden.

9. System nach Anspruch 1, bei welchem die wieder aufladbare Batterie einen zu ihr eng benachbart montierten Temperatursensor besitzt, der über das HF-Signal Sendemittel mit dem ersten Steuermittel der Sendeeinheit gekoppelt ist, wobei der Pegel der gesendeten HF-Energie proportional zu der Verringerung der Laderate der wieder aufladbaren Batterie in der Empfangseinheit reduziert werden kann, um den Energiebezug von einer die Sendeeinheit versorgenden Energiequelle zu verringern.

10. System nach Anspruch 1, bei welchem das erste Steuermittel der Sendeeinheit ein Mittel zur Steuerung des Pegels der HF-Energieübertragung von der Sendeeinheit zu der Empfangseinheit bezüglich eines oder mehrerer der folgenden Parameter aufweist: (a) Ladepegel der wieder aufladbaren Batterie, (b) gewählte Laderrate und (c) die für die Empfangseinheit gewählte Stromversorgung.

11. System nach Anspruch 1, bei welchem die Sendeeinheit ein mit dem ersten Steuermittel in Verbindung stehendes Sichtdisplay aufweist.

12. System nach Anspruch 1, bei welchem die Sendeeinheit ein mit dem ersten Steuermittel verbundenes Tastenfeld besitzt, um das Laden der wieder aufladbaren Batterie in der implantierbaren medizinischen Einrichtung zu starten und zu beenden.

13. System nach Anspruch 1, bei welchem die Sendeeinheit tragbar ist, eine Batterie beinhaltet und von einer Wechselspannungs-Energiequelle unabhängig ist.

14. System nach Anspruch 1, bei welchem die implantierbare medizinische Einrichtung aus der Gruppe gewählt ist, die besteht aus: einem Gewebestimulator, einem Arzneimittel-Zuführsystem, einem Herzschrittmachersystem und einem Kardioverter/Defibrillator.

## Revendications

1. Système médical implantable à couplage RF (10) comprenant :
une unité d'émission (12) ;
une unité de réception (14) incluant un dispositif médical implantable à commande électrique ;
la dite unité d'émission incluant des moyens d'émission d'énergie RF (64), des moyens de réception de signal RF (38) et des premier moyens de commande (26) couplés aux dits moyens d'émission d'énergie RF et aux dits moyens de réception de signal RF pour régler la quantité d'énergie RF envoyée à la dite unité de réception ;
la dite unité de réception incluant des moyens de réception d'énergie RF (60), des moyens d'émission de signal RF, une alimentation en énergie rechargeable (44) couplée aux dits moyens de réception d'énergie RF, et des deuxièmes moyens de commande (46) pour régler le courant de charge introduit dans la dite pile rechargeable couplés à la dite alimentation en énergie rechargeable, aux dits moyens de réception d'énergie RF, aux dits moyens d'émission de signal RF et au dit dispositif médical implanté ; et
des moyens de sélection de mode pour établir la dite unité d'émission pour un fonctionnement dans un des modes suivants : 1) « RF seulement », 2) « pile seulement » ou 3) « combinaison des deux ».

2. Système selon la revendication 1, dans lequel la dite unité de réception, lorsque la dite unité d'émission est établie pour fonctionner dans le dit mode « RF seulement », peut fonctionner de manière à fournir une énergie électrique au dit dispositif implantable, pourvu que la dite unité d'émission soit proche de la dite unité de réception et que la dite unité de réception détecte l'énergie RF transmise.

3. Système selon la revendication 1, dans lequel la dite unité de réception, lorsque la dite unité d'émission est établie de manière à fonctionner dans le dit mode « pile seulement », peut fonctionner périodiquement pour fournir une énergie électrique au dit dispositif implantable à partir de la dite alimentation en énergie rechargeable pendant une période d'au moins 24 heures.

4. Système selon la revendication 1, dans lequel les dits moyens d'émission d'énergie RF de la dite unité d'émission comprennent des moyens de sélection de mode pour recharger la dite pile rechargeable à un débit « rapide » ou à un débit de « maintien ».

5. Système selon la revendication 1, dans lequel la dite unité de réception, lorsque la dite unité d'émission est établie de manière à fonctionner dans le dit mode de « combinaison », peut fonctionner pour fournir une énergie électrique au dit dispositif implantable, par l'intermédiaire d'un redresseur, directement au dit dispositif médical implanté, pourvu que la dite unité d'émission soit proche de la dite unité de réception et, d'autre part, pour fournir une « charge de maintien » à la dite alimentation en énergie rechargeable.

6. Système selon la revendication 1, dans lequel la dite unité de réception comprend en outre des moyens pour mesurer le niveau de charge de la dite pile rechargeable et, lors de la détection d'une pile complètement chargée, pour renvoyer automatiquement un signal codé qui ordonne à la dite unité d'émission de « cesser » la transmission d'énergie RF.

7. Système selon la revendication 1, dans lequel la dite unité de réception comprend un boîtier en titane contenant les dits moyens de réception d'énergie RF, les dits moyens d'émission de signal RF, la dite pile rechargeable et les dits deuxièmes moyens de commande.

8. Système selon la revendication 1, dans lequel les dits moyens d'émission d'énergie RF de la dite unité d'émission sont construits de façon à émettre une énergie à une fréquence aussi basse que 10 Hz et jusqu'à 20 000 Hz au moins.

9. Système selon la revendication 1, dans lequel la dite pile rechargeable comporte un capteur de température qui est monté de façon étroitement adjacente à la dite pile et qui est couplé via les dits moyens d'émission de signal RF aux dits premiers moyens de commande de la dite unité d'émission, de sorte que le niveau de l'énergie RF transmise peut être réduit proportionnellement à la réduction du débit de charge de la pile rechargeable dans la dite unité de réception, afin de réduire la consommation d'énergie à partir d'une source d'énergie alimentant la dite unité d'émission.

10. Système selon la revendication 1, dans lequel les dits premiers moyens de commande de la dite unité d'émission comprennent des moyens de réglage du niveau de transfert d'énergie RF de l'unité d'émission à l'unité de réception en fonction d'un ou plusieurs des paramètres suivants : (a) le niveau de charge de la dite pile rechargeable, (b) le débit de charge choisi, et (c) l'alimentation en énergie sélectionnée pour la dite unité de réception.

11. Système selon la revendication 1, dans lequel la dite unité d'émission comprend un affichage visuel couplé aux dits premiers moyens de commande.

12. Système selon la revendication 1, dans lequel la dite unité d'émission comprend un clavier couplé aux dits premiers moyens de commande, comportant des touches pour démarrer et arrêter la recharge de la dite pile rechargeable dans le dispositif médical implantable.

13. Système selon la revendication 1, dans lequel la dite unité d'émission comprend une pile, de sorte que la dite unité d'émission est portable et ne dépend pas d'une source d'énergie en courant alternatif.

14. Système selon la revendication 1, dans lequel le dit dispositif médical implantable est choisi dans le groupe comprenant un stimulateur de tissu, un système d'administration de médicament, un système de stimulateur cardiaque et un régulateur / défibrillateur cardiaque.
